# EUROPÄISCHE PATENTSCHRIFT

(11) **EP 0 437 248 B1**
(45) Veröffentlichungstag und Bekanntmachung des Hinweises auf die Patenterteilung: **06.04.1994**
(21) Anmeldenummer: 91100207.9
(22) Anmeldetag: 08.01.1991
(51) Int. Cl.: A61M 25/00, A61M 25/06

(54) **Sonde zum Einführen in eine Körperhöhle**
Catheter for introducing into a body cavity
Cathéter pour introduire dans une cavité corporelle

(30) Priorität: 10.01.1990 DE 4000570
(43) Veröffentlichungstag der Anmeldung: 17.07.1991
(73) Patentinhaber: N.V. Nutricia, NL-2712 HM Zoetermeer (NL)
(72) Erfinder: Iwatschenko, Peter, W-8524 Neunkirchen (DE); Wolkenstörfer, Reinhold, W-8524 Neunkirchen (DE)
(74) Vertreter: von Hellfeld, Axel, Dr. Dipl.-Phys.

(56) Entgegenhaltungen:
- US-A- 3 358 684
- US-A- 4 253 463

## Beschreibung

Die Erfindung betrifft eine Sonde zum Einführen in eine Körperhöhle mit einem biegbaren Schlauch, der an seinem distalen Ende einen Ansatz aus einer durch Körperflüssigkeit lösbaren Substanz aufweist.

Die Schläuche solcher medizinischen Sonden bestehen aus hochmolekularen Werkstoffen, wie Kunststoff, oder auch Latex od. dgl. Der Begriff Sonde umfaßt insbesondere Katheter für medizinische Zwecke, einschließlich der "künstlichen" Ernährung, wie der perkutanen Gastrostomie etc.

An solche medizinischen Sonden werden sich widersprechende Anforderungen gestellt. Zum einen sollen sie nämlich beim Einführen in den Körper hinreichend steif sein, um eingeschoben werden zu können, und zum anderen sollen sie im eingeführten Zustand weich und flexibel sein, da sie u.U. über längere Zeitspannen im Patienten verbleiben und dort keine Störungen verursachen sollen. Mangelnde Weichheit des Schlauches kann die Bewegungsfreiheit des Patienten behindern und im Körper zu Drucknekrosen führen.

Aus der DE-C- 28 51 547 ist eine Sonde zum Einführen in Körperhöhlen bekannt, die einen Versteifungsüberzug aus in Körpersäften löslichem Material aufweist. Der Versteifungsüberzug bewirkt zunächst eine für das Einführen der Sonde günstige Versteifung derselben, löst sich dann aber in den Körpersäften auf, so daß der Schlauch des Katheters eine gute Weichheit und Biegbarkeit hat.

Aus der EP-A-238 952 ist ein Katheter zum Einführen in Körperhöhlen, insbesondere für die perkutane Gastrostomie, bekannt, der nach dem Einführen in eine Körperhöhle dort mit einem vorgegebenen Abschnitt eine spiralförmige Gestalt annimmt und sich so an der Wandung der Körperhöhle abstützt, daß das Sondenende frei in die Körperhöhle ragt und nicht an deren Wandung scheuert. Der dort beschriebene Katheter weist gemäß einer Variante ein ausklappbares Bauteil auf, das mittels eines Ansatzes aus durch Körpersäfte lösbarem Material, wie Gelatine, in Anlage an dem Schlauch des Katheters gehalten wird. Nach Auflösung der Gelatine klappen die Bauteile auf und positionieren so das Schlauchende in der Körperhöhle.

Medizinische Sonden mit einem Überzug aus durch Körpersäfte lösbarem Material sind auch in der EP-A-155 009 beschrieben. Dort ist das Schlauchende durch eine Verschlußkappe verschlossen und über der Verschlußkappe ist ein Ansatz aus durch Körpersäfte lösbarem Material angeordnet. Über diesen Ansatz wiederum ist ein weiterer Überzug aus einem Material vorgesehen, wobei das letztgenannte Material gegen Magensäure resistent ist, sich aber im Darm auflöst. Die Überzüge sind konisch zugespitzt, wobei die Zuspitzung proximal ausgerichtet ist.

Aus der gattungsbildenden US-A-3,358,684 ist eine Sonde zum Einführen in eine Körperhöhle mit einem biegbaren Schlauch bekannt, wobei der am distalen Ende der Sonde vorhandene Ansatz aus durch Körperflüssigkeit lösbarer Substanz entweder in einen distalen Endabschnitt des Schlauches ein- oder aufgepresst ist. Das distale Ende des beispielsweise aus Hart-Gelatine bestehenden Ansatzes ist, wie auch bei herkömmlichen Kanülennadeln aus Metall üblich, spitzwinklig abgeschrägt ausgeführt, um eine umlaufende, scharfe Schneidkante zu bilden, die zum Durchstechen von Körpergewebe oder einer Venenwand geeignet ist. Nach dem Durchstechen löst sich der Ansatz im Blutstrom oder einer anderen Körperflüssigkeit auf. Der Ansatz weist einen im biegbaren Schlauch mündenden Durchgangskanal auf, der nicht zur Aufnahme einer Kanüle ausgebildet ist. Damit der Ansatz aus dem in Körpersäften lösbaren Material eine zum Durchstechen von Körpergewebe ausreichende Stabilität aufweist, muß seine Mindestwanddicke größer als die der herkömmlich verwendeten Metallkanülen sein, wodurch die sich ergebende Durchtrittsöffnung größer als erwünscht ausfällt.

Durch Körpersäfte lösbare Materialien, insbesondere Gelatine, sind dem Fachmann bekannt, insbesondere aus den genannten Druckschriften.

Der Erfindung liegt die Aufgabe zugrunde, eine Sonde der gattungsgemäßen Art so weiterzubilden, daß das Einführen der Sonde in einen Patienten möglichst schonend erfolgen kann.

Erfindungsgemäß ist diese Aufgabe mit einer Sonde gelöst, die die Merkmale des Anspruchs 1 aufweist. Bei der erfindungsgemäßen Sonde wird zum Durchstechen des Körpergewebes eine herkömmliche Kanüle eingesetzt, um die sich ergebende Durchtrittsöffnung möglichst klein zu halten. Die Kanüle ist deshalb durch den Ansatz aus durch Körperflüssigkeit lösbarer Substanz schiebbar, der durch seine in distaler Richtung konische Zuspitzung eine möglichst schonende Aufweitung der Durchtrittsöffnung gewährleistet. Um die durch den Ansatz erfolgende Aufweitung der Durchtrittsöffnung so gering wie möglich zu halten, ist der maximale Außendurchmesser des Ansatzes nicht größer als der Außendurchmesser des sich daran anschließenden Schlauches. Die erfindungsgemäß am distalen Ende des Schlauches an dessen Innenwand vorgesehene, mit der Kanüle zusammenwirkende konische Anschlagsfläche sorgt dafür, daß die Kanüle in Bezug auf den Schlauch und den Ansatz genau positioniert ist und nur soweit wie nötig distal aus dem Ansatz hervorragt. Auf diese Weise wird ebenfalls zur Schonung des Patienten beigetragen, indem die Wahrscheinlichkeit einer unbeabsichtigten Verletzung von nicht zu durchstechendem Körpergewebe herabgesetzt ist.

Der zugespitzte Ansatz aus einem durch Körperflüssigkeiten lösbaren Material erlaubt ein einfaches Einführen der Sonde in den Körper. Ist die Sonde im Körper plaziert, löst sich der Ansatz auf, so daß gewährleistet ist, daß das distale Sondenende nicht an einer Wandung der Körperhöhle scheuert oder gar sich in einer Faltung dieser Wandung verfängt. Eine solche Gefahr besteht insbesondere beim Einführen eines Katheters in den Magen.

Die Erfindung betrifft somit insbesondere eine Verbesserung des Gegenstandes der oben genannten EP-A-238 952, deren Offenbarungsgehalt hier ausdrücklich eingeschlossen ist.

Gemäß einer bevorzugten Ausgestaltung der Erfindung ist der Schlauch des Katheters an seiner distalen Stirnfläche mit einer zentralen Öffnung versehen, welche von dem Ansatz aus durch Körpersäfte lösbarem Material überragt wird. Nachdem der Ansatz aufgelöst ist, liegt das Schlauchende frei, so daß Nahrung, Medikamente etc. ungehindert in die Körperhöhle eingeführt werden können.

Das Einführen der Sonde in die Körperhöhle wird gemäß einer besonderen Ausgestaltung der Erfindung dann besonders erleichtert, wenn der Schlauch oder ein mit ihm verbundenes Spitzenteil am distalen Ende einen verjüngten Abschnitt aufweist, auf dem der Ansatz angeordnet ist.

Nachfolgend wird ein Ausführungsbeispiel der Erfindung anhand der Zeichnung näher erläutert. Es zeigt:
- Fig. 1: das distale Ende einer Sonde;
- Fig. 2: die Sonde gemäß Fig. 1 ohne Kanüle und
- Fig. 3: die Sonde gemäß Fig. 1 und 2 ohne Kanüle und Ansatz.

Die in den Figuren gezeigte Sonde dient als Katheter, insbesondere für die perkutane Gastrostomie.

In den Figuren ist nur das distale Ende des Katheters dargestellt. Die distale Richtung ist durch einen Pfeil R angedeutet.

Ein Schlauch 10 aus biegsamen Material weist an seinem distalen Ende einen Ansatz 12 aus durch Körpersäfte lösbarem Material, wie Gelatine, auf. Eine Kanüle 14 durchragt zentrisch den Schlauch 10 und den Ansatz 12.

Die Kanüle 14 ist beispielsweise für die perkutane Gastrostomie (EP 238 952) erforderlich, um den Katheter durch die Bauchdecke und die Magenwand in das Magenlumen einzuführen. Hierzu ist die Kanüle 14 am distalen Ende unter Bildung einer Spitze 16 angeschliffen. Die Kanüle 14 ist hohl, so daß sich an der Spitze 16 beim Anschleifen eine Öffnung 18 ergibt.

Der Ansatz 12 aus durch Körpersäfte lösbarem Material ist in distaler Richtung R konisch zugespitzt geformt. Die konischen Flächen 20 des Ansatzes 12 erleichtern das Einführen des Katheters. Der Ansatz 12 liegt dicht um die Kanüle 14, wobei das spitze Ende 22 des Ansatzes 12 sich an die Außenwand der Kanüle 14 anschmiegt.

Der Schlauch 10 des Katheters weist an seinem distalen Ende eine zentrale Öffnung 24 auf, deren Durchmesser etwas größer ist als der Durchmesser der Kanüle 14.

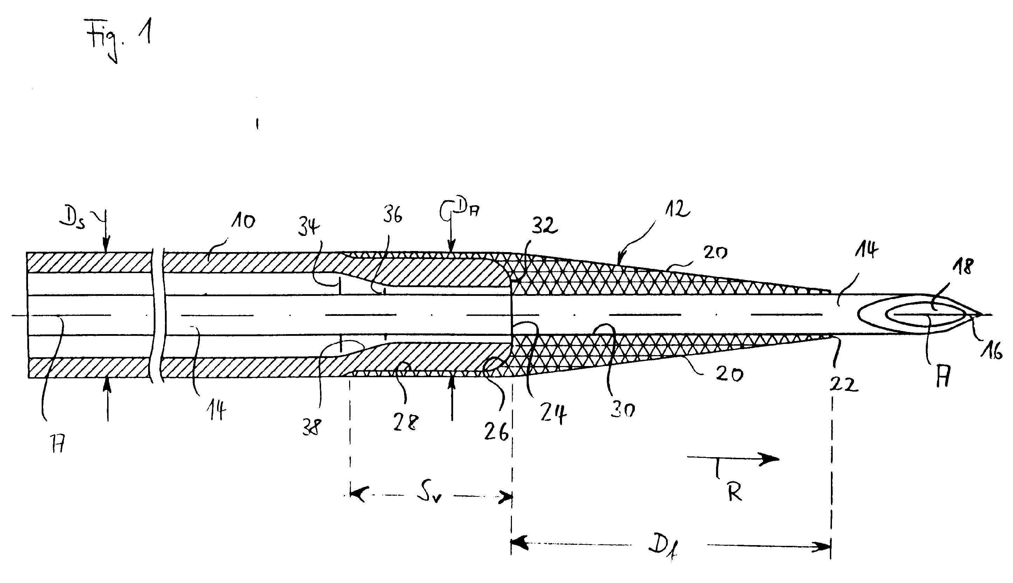

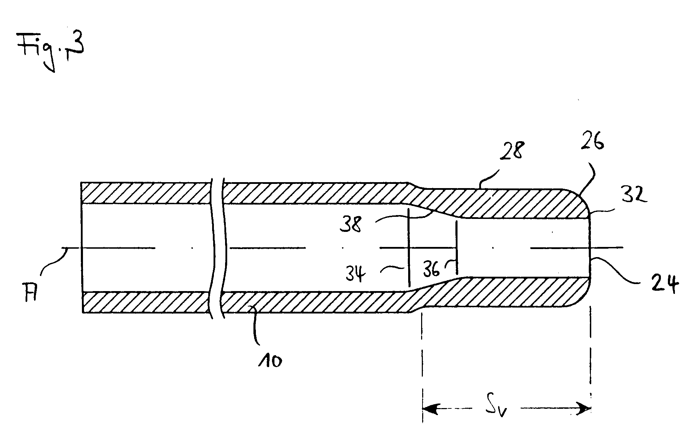

Am distalen Ende weist der Schlauch 10 einen Abschnitt Sᵥ mit reduziertem Außendurchmesser auf. Auf der Mantelfläche 28 des Schlauches 10 im Abschnitt Sᵥ mit verjüngtem Durchmesser sitzt ein Abschnitt des Ansatzes 12 gemäß Fig. 1. Der Ansatz 12 umschließt dicht die Mantelfläche 28 des Schlauches 10 sowie die distale Stirnfläche 32 des Schlauches 10. Die Stirnfläche 32 ist am distalen Ende im Bereich der Kanten 26 abgerundet (siehe auch Fig. 3).

Der maximale Außendurchmesser D_{A} des Ansatzes 12 entspricht etwa dem Außendurchmesser D_{S} des Schlauches 10, so daß keine Kanten oder Versprünge gebildet sind. Die Anordnung ist insgesamt so schlank als möglich.

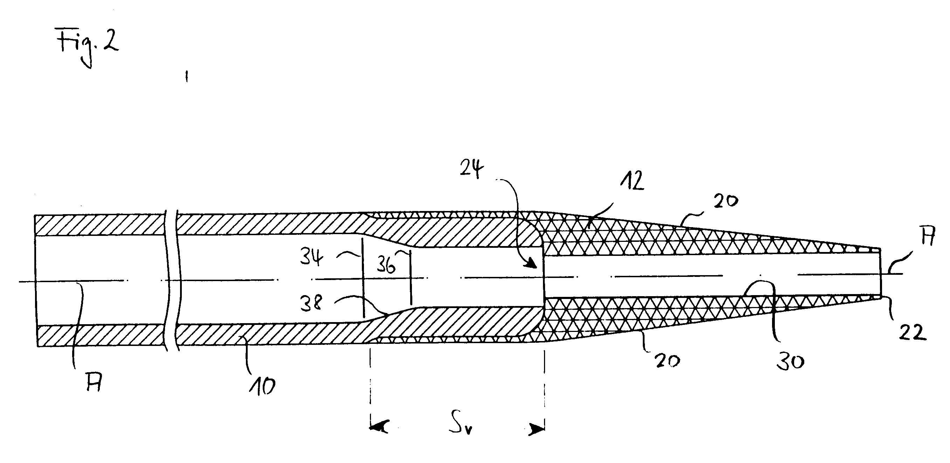

Wie in Fig. 1 bis 3 durch Striche angedeutet ist, weist die Kanüle 14 Anschläge 34, 36 auf, die mit einer konischen Anschlagsfläche 38 zusammenwirken, die am distalen Ende des Schlauches 10 in dessen Innenwand ausgebildet ist. Durch die Anschläge 34, 36 bzw. 38 ist die Kanüle 14 genau in Bezug auf den Schlauch 10 und den Ansatz 12 positioniert. Sie ragt mit der Spitze 16 aus der Spitze 22 des Ansatzes 12.

Der Ansatz 12 überragt in distaler Richtung R die Stirnfläche 32 des Schlauches 10. Die Distanz D_{f}, um welche der Ansatz 12 in distaler Richtung über die Stirnfläche 32 des Schlauches 10 vorsteht, beträgt das Ein- bis Fünffache, vorzugsweise das Zwei- bis Dreifache des Außendurchmesser D_{S} des Schlauches 10.

Nach dem Einführen des Katheters wird die Kanüle 14 zurückgezogen und der Ansatz 12 löst sich im Körper auf. Dadurch ist gewährleistet, daß die Stirnfläche 32 sowie die Öfffnung 24 im Schlauch 10 nicht direkt an einer Wandung der Körperhöhle anliegen oder gar in einer Falte der Wandung gefangen sind.

## Patentansprüche

1. Sonde zum Einführen in eine Körperhöhle mit einem biegbaren Schlauch (10), der an seinem distalen Ende einen Ansatz (12) aus einer durch Körperflüssigkeit lösbaren Substanz aufweist,
dadurch **gekennzeichnet,** daß
- der Ansatz (12) konisch in distaler Richtung (R) zugespitzt ist und einen Kanal (30) zur Aufnahme einer durch den Ansatz hindurch schiebbaren Kanüle (14) aufweist,
- der maximale Außendurchmesser (D_{A}) des Ansatzes (12) nicht größer ist als der Außendurchmesser (D_{S}) des Schlauches (10), und
- am distalen Ende des Schlauches (10) an dessen Innenwand eine mit der Kanüle (14) zusammenwirkende konische Anschlagsfläche (38) ausgebildet ist.

2. Sonde nach Anspruch 1,
dadurch **gekennzeichnet,** daß der Schlauch (10) an seiner distalen Stirnfläche (32) eine zentrale Öffnung (24) aufweist, die der Ansatz (12) in distaler Richtung überragt.

3. Sonde nach einem der vorhergehenden Ansprüche,
dadurch **gekennzeichnet,** daß der Schlauch (10) oder ein mit ihm verbundenes Spitzenteil am distalen Ende einen verjüngten Abschnitt (Sᵥ) aufweist, auf dem der Ansatz (12) angeordnet ist.

## Claims

1. A probe to be introduced into a body cavity, comprising a pliable hose (10) which has, at its distal end, an attachment (12) made of a substance which is dissoluble by body fluid,
**characterized** in that
- the attachment (12) tapers conically in distal direction (R) and has a passage (30) for reception of a cannula (14) which is adapted to be pushed through the attachment,
- the maximum outer diameter (D_{A}) of the attachment (12) is not greater than the outer diameter (D_{S}) of the hose (10), and
- the inside wall of the hose (10) is formed, at the distal end of the hose, with a conical abutment surface (38) which cooperates with the cannula (14).

2. The probe as claimed in claim 1, characterized in that the hose (10) has a central opening (24) at its distal end surface (32) and the attachment (12) projects beyond the same in distal direction.

3. The probe as claimed in any one of the preceding claims, characterized in that the hose (10), or a tip element connected to it, includes a narrowed portion (Sᵥ) at the distal end on which portion the attachment (12) is arranged.

## Revendications

1. Sonde destinée à être introduite dans une cavité du corps, comprenant un tuyau flexible (10) qui présente à son extrémité distale un tambour (12) fait d'une substance soluble dans les liquides corporels,
caractérisée en ce que
- l'embout (12) est effilé en cône dans la direction distale (R) et présente un canal (30) destiné à recevoir une canule (14) qui peut être enfilée à travers l'embout ;
- le diamètre extérieur maximum (D_{A}) de l'embout (12) n'est pas supérieur au diamètre extérieur (D_{S}) du tuyau (10) ; et
- une surface de butée conique (38) coopérant avec la canule (14) est formée à l'extrémité distale du tuyau (10), au droit de sa paroi inférieure.

2. Sonde selon la revendication 1,
caractérisée en ce que le tuyau (10) présente sur son extrémité frontale distale (32), une ouverture centrale (24) que l'embout (12) dépasse dans la direction distale.

3. Sonde selon une des revendications précédentes,
caractérisée en ce que le tuyau (10), ou bien une partie pointe solidaire de ce tuyau, présente à l'extrémité distale un segment rétréci (Sᵥ) sur lequel l'embout (12) est monté.
